(19) Europäisches Patentamt / European Patent Office / Office européen des brevets

(11) **EP 2 014 300 B1**

(12) **EUROPEAN PATENT SPECIFICATION**

(45) Date of publication and mention
of the grant of the patent:
**12.01.2011 Bulletin 2011/02**

(51) Int Cl.:
*A61K 39/00* (2006.01)   *A61K 45/08* (2006.01)
*A61P 35/00* (2006.01)   *A61P 35/02* (2006.01)

(21) Application number: **08013828.2**

(22) Date of filing: **28.06.2002**

(54) **Cancer vaccine comprising a cancer antigen based on the product of a tumor suppressor gene WT1 and a cationic liposome**

Krebsimpfstoff mit einem Krebs-Antigen auf Grundlage des Produkts aus dem Tumorunterdrückungsgen WT1 und einem kationischen Liposom

Vaccin contre le cancer comprenant un antigène du cancer basé sur le produit d'un gène wt1 suppresseur de tumeur et d'un lipomose cationique

(84) Designated Contracting States:
**AT BE CH CY DE DK ES FI FR GB GR IE IT LI LU MC NL PT SE TR**

(30) Priority: **29.06.2001 JP 2001199449**

(43) Date of publication of application:
**14.01.2009 Bulletin 2009/03**

(62) Document number(s) of the earlier application(s) in accordance with Art. 76 EPC:
**02738876.8 / 1 410 804**

(73) Proprietors:
• **Chugai Seiyaku Kabushiki Kaisha Kita-ku Tokyo 115-8543 (JP)**
• **Mayumi, Tadanori Koube-shi, Hyogo 655-0041 (JP)**
• **Sugiyama, Haruo Minoo-shi, Osaka 562-0036 (JP)**

(72) Inventors:
• **Mayumi, Tadanori Chou-Ku Kobe 650-8586 (JP)**
• **Sugiyama, Haruo Minoo-shi Osaka 562-0036 (JP)**
• **Ohsugi, Yoshiyuki Tokyo 115-8543 (JP)**

(74) Representative: **Holliday, Louise Caroline D Young & Co LLP 120 Holborn London EC1N 2DY (GB)**

(56) References cited:
EP-A1- 1 103 564   WO-A-00/26249
WO-A2-00/18795   WO-A2-01/25273

• FAN ZHOU ET AL: "DELIVERY OF PROTEIN ANTIGEN TO THE MAJOR HISTOCOMPATIBILITY COMPLEXCLASS I-RESTRICTED ANTIGEN PRESENTATION PATHWAY" JOURNAL OF DRUG TARGETING, HARWOOD ACADEMIC PUBLISHERS GMBH, DE, vol. 3, no. 2, January 1995 (1995-01), pages 91-109, XP000601245 ISSN: 1061-186X
• NAIR S ET AL: "CLASS I RESTRICTED CTL RECOGNITION OF A SOLUBLE PROTEIN DELIVERED BY LIPOSOMES CONTAINING LIPOPHILIC POLYLYSINES" JOURNAL OF IMMUNOLOGICAL METHODS, ELSEVIER SCIENCE PUBLISHERS B.V.,AMSTERDAM, NL, vol. 152, no. 2, 1992, pages 237-243, XP000608281 ISSN: 0022-1759

EP 2 014 300 B1

**Description**

Field of the Invention

The present invention relates to a cancer vaccine comprising a cancer antigen based on the product of a tumor suppressor gene WT1 of Wilms tumor and lipofectin. This cancer vaccine is useful as an anti-cancer vaccine for blood cancers such as leukemia, myelodysplastic syndrome, multiple myeloma and malignant lymphoma, or solid cancers such as gastric cancer, colon cancer, lung cancer, breast cancer, germ cell cancer, liver cancer, skin cancer, bladder cancer, prostatic cancer, uterine cancer, cervical cancer and ovarian cancer, as well as all other cancers that express WT1.

Background Art

Immunological mechanisms for eliminating foreign substances generally comprise the humoral immunity which involves macrophages that recognize antigen so as to function as antigen presenting cells, helper T cells that recognize the antigen presentation by said macrophages and produce various lymphokines so as to activate other T cells etc., and B lymphocytes, etc., that differentiate into antibody-producing cells by the action of said lymphokines; and the cellular immunity in which killer T cells differentiated by antigen presentation attack and destroy target cells.

At present, cancer immunity is mainly derived from cellular immunity which involves killer T cells. In killer T cell-mediated cancer immunity, precursor T cells that recognized cancer antigen presented in the form of a complex with the major histocompatibility complex (MHC) class I differentiate and grow to produce killer T cells, which attack and destroy cancer cells. At this time, cancer cells have presented the complex of the MHC class I antigen and cancer antigen on the cell surface, which becomes the target for killer T cells (Curr. Opin. Immuno. 5:709, 1993; Curr. Opin. Immunol. 5:719, 1993; Cell 82:13, 1995; Immunol. Rev. 146:167, 1995).

The above cancer antigen presented on the target cancer cells by MHC class I antigen is believed to be a peptide composed of about 8-12 amino acids produced as a result of processing by intracellular protease of antigen proteins synthesized in the cancer cells (Curr. Opin. Immunol. 5:709, 1993; Curr. Opin. Immunol. 5:719, 1993; Cell 82: 13, 1995; Immunol. Rev. 146:167, 1995).

The tumor suppressor gene WT1 (WT1 gene) of Wilms tumor was isolated from chromosome 11p13 as one of the causative genes for Wilms tumor based on the analysis of the WAGR syndrome that is accompanied by Wilms tumor, aniridia, urogenital abnormality, mental retardation etc. (Gessler, M. et al., Nature, Vol. 343, pp. 774-778, 1990), and its genomic DNA is about 50 kb comprising ten exons and its cDNA is about 3 kb. The amino acid sequence deduced from the cDNA is as set forth in SEQ ID NO: 1 (Mol. Cell. Biol. 11:1707, 1991).

The WT1 gene is highly expressed in human leukemia, and the treatment of leukemic cells with a WT1 antisense oligomer leads to the suppression of the cell growth (Japanese Unexamined Patent Publication (Kokai) No. 9-104627), which suggests that the WT1 gene is acting on the growth of leukemic cells in a facilitative manner. Furthermore, the WT1 gene has also been highly expressed in solid cancers such as gastric cancer, colon cancer, lung cancer, breast cancer, germ cell cancer, liver cancer, skin cancer, bladder cancer, prostatic cancer, uterine cancer, cervical cancer and ovarian cancer (Japanese Patent Application No. 9-191635), and the WT1 gene was found to be a new tumor marker for leukemia and solid cancers.

Thus, it is expected that the administration of a peptide having about 8-12 amino acids comprising a portion of expression products of the WT1 gene could serve as a cancer vaccine against the above range of cancers. However, the administration of such a peptide as it is cannot serve as a cancer vaccine. This is because it is expected that the peptide administered cannot be effectively delivered to the major histocompatibility complex class I on the antigen-presenting cells.

Lipofectin, a cationic liposome, is a 1:1 mixture of an artificial lipid N-[1-(2,3-dioleyloxy)propyl]-N,N,N-trimethylammonium chloride (DOTMA) and a phospholipid dioleoylphosphatidylethanolamine (DOPE), and attracted attention as a nonviral carrier for introducing genes. Subsequently, attention was given to the fact that it can serve to deliver peptide antigens to the major histocompatibility complex class I on the antigen-presenting cells (Rinsho Menneki 34(6): 842-847, 2000). However, the degree of versatility of cationic liposomes as carriers for peptide antigens is unknown, and it is not known either whether they can serve as carriers for cancer antigen peptides comprising fragments of expression products of the tumor suppressor gene WT1 gene.

Disclosure of the Invention

Thus, the present invention provides a novel cancer vaccine comprising a cancer antigen peptide derived from a WT1 gene expression product and a substance useful as a carrier therefor.

After intensive and extensive research in order to solve the above problems, the present inventors have confirmed that, in the amino acid sequence of expression product of the WT1 gene, a polypeptide comprising 7-30 contiguous amino acids containing at least one amino acid that is estimated to serve as an anchor amino acid serves as a cancer antigen in the binding with the mouse and human MHC class I and MHC class II, and that cationic liposomes such as lipofectin are useful as carriers for this peptide antigen and, thereby, have completed the present invention.

Thus, the present invention provides a cancer vaccine comprising the cancer antigen peptide comprising the amino acid sequence Arg Met Phe Pro Asn Ala Pro Tyr Leu (SEQ ID NO: 5)and a cationic liposome.

The cancer may be leukemia, myelodysplastic syndrome, malignant lymphoma, multiple myeloma, gastric cancer, colon cancer, lung cancer, breast cancer, germ cell cancer, liver cancer, skin cancer, bladder cancer, prostatic cancer, uterine cancer, cervical cancer or ovarian cancer.

The cationic liposome may be lipofectin.

The present invention also provides the use of the cancer antigen peptide comprising the amino acid sequence Arg Met Phe Pro Asn Ala Pro Tyr Leu (SEQ ID NO: 5) and a cationic liposome for the production of cancer vaccine.

The cancer may be leukemia, myelodysplastic syndrome, malignant lymphoma, multiple myeloma, gastric cancer, colon cancer, lung cancer, breast cancer, germ cell cancer, liver cancer, skin cancer, bladder cancer, prostatic cancer, uterine cancer, cervical cancer or ovarian cancer.

The cationic liposome may be a liposome comprising N-[1-(2,3-dioleyloxy)propyl]-N,N,N-trimethylammonium chloride, N-[1-(2,3-dioleyloxy)propyl]-N,N,N-trimethylammonium methyl sulfate or dioctadecylamide-glycylspermine; or a mixture thereof with a neutral lipid.

The cationic liposome may be lipofectin.

The present invention also provides the cancer antigen peptide comprising the amino acid sequence Arg Met Phe Pro Asn Ala Pro Tyr Leu (SEQ ID NO: 5) and a cationic liposome for treating patents with cancer.

The cancer may be leukemia, myelodysplastic syndrome, malignant lymphoma, multiple myeloma, gastric cancer, colon cancer, lung cancer, breast cancer, germ cell cancer, liver cancer, skin cancer, bladder cancer, prostatic cancer, uterine cancer, cervical cancer or ovarian cancer.

The cationic liposome may be a liposome comprising N-[1-(2,3-dioleyloxy)propyl]-N,N,N-trimethylammonium chloride, N-[1-(2,3-dioleyloxy)propyl]-N,N,N-trimethylammonium methyl sulfate or dioctadecylamide-glycylspermine; or a mixture thereof with a neutral lipid.

The cationic liposome may be lipofectin.

Brief Explanation of the Drawings

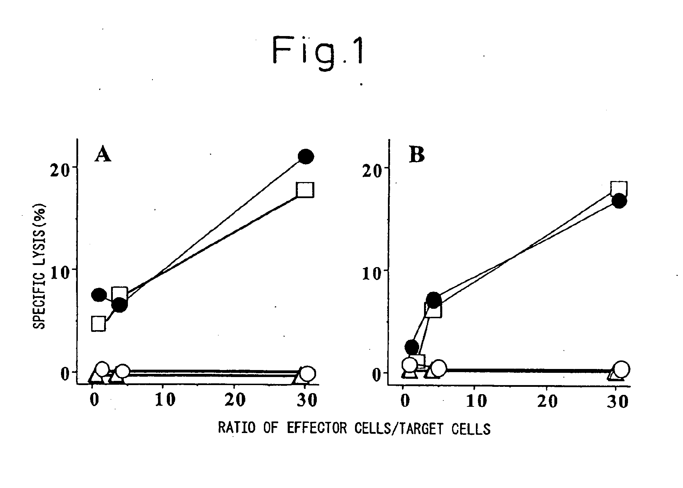

In Fig. 1, A is a graph that compares the ability of inducing cytotoxic T cells of a mixture (closed circle) of a cancer antigen peptide $D^b$ 126 and lipofectin (LPF), a lipopolysaccharide-blast (open square) pulsed with $D^b$ 126, lipofectin alone (open triangle) and the cancer antigen peptide $D^b$ 126 alone (open circle) using (3) RNAS cells and (4) RNAS cells stimulated with the cancer antigen peptide $D^b$ 126, and B is a graph of the result in which tests similar to the above A were carried out using (1) C1498 cells and (2) WT1 gene-introduced C1498 cells. A indicates that the combination of the cancer antigen peptide $D^b$ 126 and lipofectin has an activity of inducing cytotoxic T cells, and B indicates that the activity thereof is WT1-specific.

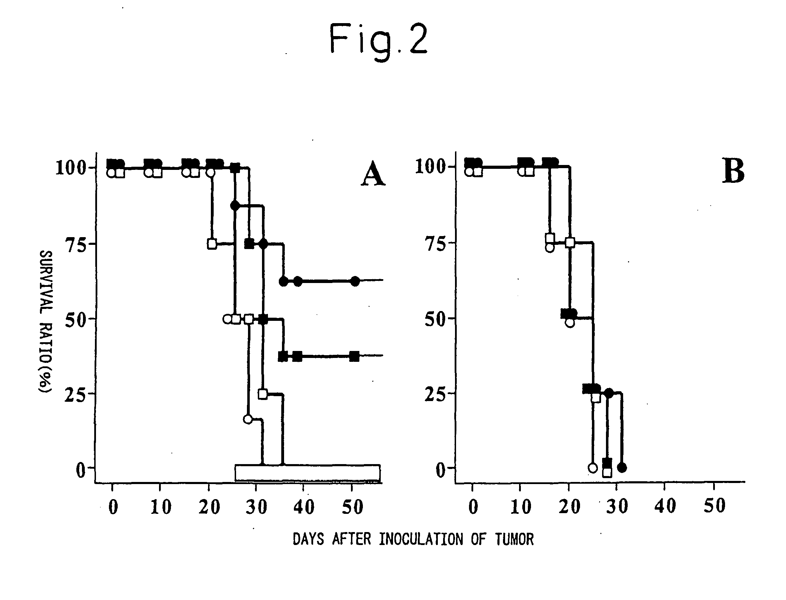

In Fig. 2, A is a graph that shows the effect of lipofectin as an adjuvant (carrier) for the anti-cancer effect of the peptide $D^b$ 126 using WT1 gene-introduced C1498 cells, and B is a graph that shows the result of similar tests using C1498 cells. Signs that indicate the test substances are the same as in Fig. 1. A indicates that lipofectin is effective as an adjuvant (carrier) for the cancer antigen peptide $D^b$ 126, and the comparison of A and B shows that the anti-cancer effect is WT1-specific.

Embodiment for Carrying Out the Invention

As a basis for designing cancer antigen peptides, $K^b$ and $D^b$ of mouse MHC class I as well as A0201 of human HLA were selected, and peptides estimated to have a high affinity with them were selected.

Based on the description in Immunogenetics 41:178-228 (1995), Phe and Try at position 5 as well as Leu and Met etc. at position 8 are expected to be the anchor amino acids for binding to $K^b$, and Asn at position 5 as well as Met and Ile etc. at position 9 are expected to be the anchor amino acids for binding to $D^b$.

It is also known that the size of cancer antigen peptides presented on the surface of cancer cells by MHC class I is about 8-12 amino acids. Thus, the cancer antigen peptide of the present invention comprises the amino acid sequence Arg Met Phe Pro Asn Ala Pro Tyr Leu (SEQ ID NO: 5)

In the above sequence, the underlined amino acids are those that are thought to serve as anchors.

All sequences tested have strong to moderate binding affinities (Kd values) for $K^b$ or $D^b$, and the $D^b$ 126 peptide of the present invention having the highest binding affinity was used in the following experiments.

For humans, based on the description in Immunogenetics 41:178-228 (1995), Leu and Met at position 2 from the N-terminal and Val and Leu at position 9 from the N-terminal are expected to be anchor amino acids for binding to human HLA-A0201. Thus, from among the amino acid sequence of human WT1 protein (Mol. Cell. Biol. 11:1707-1712, 1991) (SEQ ID NO: 2), the following two peptides:

$D^b$ 126 Arg <u>Met</u> Phe Pro Asn Ala Pro Tyr <u>Leu</u> (SEQ ID NO: 5)
(the same as $D^b$ 126 in mice)
WH 187 Ser <u>Leu</u> Gly Glu Gln Gln Tyr Ser <u>Val</u> (SEQ ID NO: 8)
(the underlined are anchor amino acids)

comprising nine amino acids are mentioned as complying with the above condition.

As a cationic liposome, there can be mentioned a liposome comprising N-[1-(2,3-dioleyloxy)propyl]-N,N,N-trimethylammonium chloride (DOTMA), N-[1-(2,3-dioleyloxy)propyl]-N,N,N-trimethylammonium methyl sulfate (DOTAP) or dioctadecylamide-glycylspermine (DOGS), or mixtures thereof with a neutral lipid.

As a neutral lipid, there can be mentioned, for example, licithin, lysolecithin, sphingomyelin, phosphatidic acid, phosphatidylethanolamine, and dioleoylphosphatidylethanolamine (DOPE). As an example of mixtures, there can be mentioned lipofectin which is a 1:1 mixture of an artificial lipid N-[1-(2,3-dioleyloxy)propyl]-N,N,N-trimethylammonium chloride (DOTMA) and a phospholipid dioleoylphosphatidylethanolamine (DOPE).

The cancer vaccine of the present invention can be used for the prevention or treatment of cancers that are accompanied by increased levels of the WT1 gene expression, for example blood cancers such as leukemia, myelodysplastic syndrome, multiple myeloma and malignant lymphoma, and solid cancers such as gastric cancer, colon cancer, lung cancer, breast cancer, germ cell cancer, liver cancer, skin cancer, bladder cancer, prostatic cancer, uterine cancer, cervical cancer and ovarian cancer. This vaccine can be administered by oral administration, or parenteral administration such as intraperitoneal, cutaneous, dermal, intramuscular, intravenous, and nasal administration.

The dosage of the cancer vaccine of the present invention is generally 0.1 $\mu$g to 1 mg/kg per day.

Examples

The usefulness of the cancer vaccine of the present invention will now be explained with reference to Examples.

<u>Example 1.</u>

<u>Preparation of lipopolysaccharide-blast (LPS-blast)</u>

From C57BL/6 mice, spleen cells were recovered, and the cells were incubated for 3 days in a complete RPMI medium containing lipopolysaccharide (LPS) (10 $\mu$g/ml). After washing, the cells were incubated in a complete RPMI medium containing the cancer antigen peptide $D^b$ 126 (1 $\mu$M) and ovalbumin (OVA) (100 $\mu$g/ml). After washing, the cells were suspended in 2 ml Hanks' balanced salt solution (HBSS) which was set as the lipopolysaccharide-blast (LPS-blast).

<u>Evaluation of the ability of inducing cytotoxic T cells (CTL)</u>

C57BL/6 mice were immunized three times weekly by subcutaneous administration, to the back thereof, of a mixture of the cancer antigen peptide $D^b$ 126 and lipofectin (LPF) (mixed at a 1:2 weight ratio of $D^b$ 126 and LPF), and as a positive control by the intraperitoneal administration of lipopolysaccharide-blast (LPS-blast) (1 ml/mouse). Ten days after the final immunization, spleen cells were recovered and set as effector cells. Spleen cells stimulated with the cancer antigen peptide $D^b$ 126 (1 $\mu$M, 2 hours, 37°C, 5% CO2) were washed with HBSS to obtain stimulator cells.

The above effector cells (5 x 106 cells/well) and the above stimulator cells (2.5 x 106 cells/well) were mixed, and then subjected to lymphocyte-lymphocyte mixed culture for the in vitro second challenge of the cytotoxic T cells. Five days later, cytotoxic T cells were recovered. (1) C1498 cells, (2) C1498 cells that have introduced therein and express the WT1 gene (C1498muWT1), (3) RMA-S cells, and (4) RMA-S cells stimulated with the cancer antigen $D^b$ 126 (1 $\mu$M, treated for 1 hour at 5% CO2) ($D^b$ 126-pulsed RMA-S), each labelled with $Na_2{}^{51}CrO_4$ (0.56 MBq/$10^6$ cells, treated for 1 hour at 37°C and 5% CO2), were plated as the target cells onto 96-well microtiter plates ($10^4$ cells/well), to which cytotoxic T cells prepared as above were plated. Effector cells were added thereto, cultured for 4 hours, and the radioactivity of $^{51}$Cr liberated into the supernatant was counted. Cytotoxic activity was calculated according to the following equation:

$$\text{Cell lysis (\%)} = \frac{(\text{experimental release} - \text{spontaneous release})}{(\text{maximum release} - \text{spontaneous release})} \times 100$$

Results

First, in order to examine whether or not the induced cytotoxic T cells are specific for the cancer antigen peptide $D^b$ 126, the above (3) RMA-S cells and (4) $D^b$ 126-pulsed RMA-S cells were used as the target cells, and as a result the induction of cytotoxic T cells specific for the cancer antigen peptide $D^b$ 126 was confirmed (Fig. 1, A). Furthermore, in order to examine whether or not the induced cytotoxic T cells specifically damage WT1-expressing cells, the above (1) C1498 cells and (2) the WT1 gene-introduced cells, C1498muWT1, were used, and as a result the induction of WT1-specific CTL was confirmed (Fig. 1, B).

Example 2.

Cancer antigen-specific anti-tumor effect when lipofectin (LPF) was used as the cancer vaccine carrier

Since Example 1 has shown that cytotoxic T cells are effectively induced by using lipofectin (LPF) as an adjuvant for the cancer antigen peptide $D^b$ 126, cancer antigen-specific anti-tumor effect when immunized using lipofectin as an adjuvant (carrier) was examined for the purpose of further confirming the usefulness of lipofectin (LPF) as an adjuvant for cancer vaccines.

As the tumor model, WT1 gene-introduced C1498 cells (C1498muWT1 cells) were used; as the immunization animal, C57BL/6 mice were used; and as the model cancer antigen, the peptide $D^b$ 126 was used. Thus, C57BL/6 mice were immunized three times weekly by subcutaneous administration, to the back thereof, of the same mixture as in Example 1 of the cancer antigen peptide $D^b$ 126 and lipofectin (LPF) (10 nmol/mouse), or by the intraperitoneal administration of lipopolysaccharide-blast (LPS-blast) (1 ml), and one week after the final immunization C1498muWT1 cells or C1498 cells were intraperitoneally transplanted at an amount of $2 \times 10^6$ cells/100 ml. The effect of tumor vaccine was determined daily, and was evaluated by calculating tumor size using the following equation:

$$[\text{Tumor size}] = [(\text{long diameter}) \times (\text{short diameter})^2]^{1/3}$$

In each group, the experiment was terminated when tumor size reached 20 mm.

Results

The evaluation of lipofectin (LPF) as an adjuvant (carrier) for cancer vaccine was carried out using WT1 as the model tumor antigen and WT1 gene-introduced cells (C1498muWT1 cells) as the model tumor, and using, as an index, resistance against C1498muWT1 cells when the peptide $D^b$ 126/lipofectin (LPF) mixture was used for immunization. As a result, when the peptide $D^b$ 126/lipofectin (LPF) mixture was used for immunization, complete rejection was observed in three out of eight cases (Fig. 2, A).

Furthermore, in order to confirm that this anti-tumor effect is WT1-specific, a similar study was carried out using C1498 cells that are not expressing WT1. As a result, there were no differences seen from the non-immunized group in any of the cases in which (a) peptide $D^b$ 126/lipofectin (LPF) mixture, (b) free peptide $D^b$ 126, and (c) lipopolysaccharide-blast (LPS-blast) were immunized (Fig. 2, B). Therefore, it was confirmed that the above anti-tumor effect is WT1-specific.

**Claims**

1. A cancer vaccine comprising the cancer antigen peptide comprising the amino acid sequence Arg Met Phe Pro Asn Ala Pro Tyr Leu (SEQ ID NO: 5)and a cationic liposome.

2. The cancer vaccine according to claim 1 wherein said cancer is leukemia, myelodysplastic syndrome, malignant lymphoma, multiple myeloma, gastric cancer, colon cancer, lung cancer, breast cancer, germ cell cancer, liver cancer, skin cancer, bladder cancer, prostatic cancer, uterine cancer, cervical cancer or ovarian cancer.

3. The cancer vaccine according to claim 1 or 2 wherein said cationic liposome is a liposome comprising N-[1-(2,3-dioleyloxy)propyl]-N,N,N-trimethylammonium chloride, N-[1-(2,3-dioleyloxy)propyl]-N,N,N-trimethylammonium methyl sulfate or dioctadecylamide-glycylspermine; or a mixture thereof with a neutral lipid.

4. The cancer vaccine according to claim 3 wherein said cationic liposome is lipofectin.

5. The use of the cancer antigen peptide comprising the amino acid sequence Arg Met Phe Pro Asn Ala Pro Tyr Leu (SEQ ID NO: 5) and a cationic liposome for the production of cancer vaccine.

6. The use according to claim 5 wherein said cancer is leukemia, myelodysplastic syndrome, malignant lymphoma, multiple myeloma, gastric cancer, colon cancer, lung cancer, breast cancer, germ cell cancer, liver cancer, skin cancer, bladder cancer, prostatic cancer, uterine cancer, cervical cancer or ovarian cancer.

7. The use according to claim 6 wherein said cationic liposome is a liposome comprising N-[1-(2,3-dioleyloxy)propyl]-N, N,N-trimethylammonium chloride, N-[1-(2,3-dioleyloxy)propyl]-N,N,N-trimethylammonium methyl sulfate or dioctadecylamide-glycylspermine; or a mixture thereof with a neutral lipid.

8. The use according to claim 7 wherein said cationic liposome is lipofectin.

9. The cancer antigen peptide comprising the amino acid sequence Arg Met Phe Pro Asn Ala Pro Tyr Leu (SEQ ID NO: 5) and a cationic liposome for treating patents with cancer.

10. The cancer antigen peptide and cationic liposome according to claim 9 wherein said cancer is leukemia, myelodysplastic syndrome, malignant lymphoma, multiple myeloma, gastric cancer, colon cancer, lung cancer, breast cancer, germ cell cancer, liver cancer, skin cancer, bladder cancer, prostatic cancer, uterine cancer, cervical cancer or ovarian cancer.

11. The cancer antigen peptide and cationic liposome according to any of claim 9 or 10 wherein said cationic liposome is a liposome comprising N-[1-(2,3-dioleyloxy)propyl]-N,N,N-trimethylammonium chloride, N-[1-(2,3-dioleyloxy)propyl]-N,N,N-trimethylammonium methyl sulfate or dioctadecylamide-glycylspermine; or a mixture thereof with a neutral lipid.

12. The cancer antigen peptide and cationic liposome according to claim 11 wherein said cationic liposome is lipofectin.

**Patentansprüche**

1. Krebsimpfstoff, umfassend das Krebsantigenpeptid, welches die Aminosäuresequenz Arg Met Phe Pro Asn Ala Pro Tyr Leu (SEQ ID Nr. 5) umfasst, und ein kationisches Liposom.

2. Krebsimpfstoff nach Anspruch 1, wobei der Krebs Leukämie, myelodysplastisches Syndrom, malingnes Lymphom, multiples Myelom, Magenkrebs, Kolonkrebs, Lungenkrebs, Brustkrebs, Keimzellenkrebs, Leberkrebs, Hautkrebs, Blasenkrebs, Prostatakrebs, Gebärmutterkrebs, Gebärmutterhalskrebs oder Eierstoakkrebs ist.

3. Krebsimpfstoff nach Anspruch 1 oder 2, wobei das kationische Liposom ein Liposom ist, welches N-[1-(2,3-Dioleyloxy)propyl]-N,N,N-trimethylammoniumchlorid, N-[1-(2,3-Dioleyloxy)propyl]-N,N,N-trimethylammoniummethylsulfat oder Dioctadecylamid-glycylspermin oder ein Gemisch davon mit einem neutralen Lipid umfasst.

4. Krebsimpfstoff nach Anspruch 3, wobei das kationische Liposom Lipofektin ist.

5. Verwendung des Krebsantigenpeptids, umfassend die Aminosäuresequenz Arg Met Phe Pro Asn Ala Pro Tyr Leu (SEQ ID Nr. 5) und ein kationisches Liposom für die Herstellung eines Krebsimpfstoffs.

6. Verwendung nach Anspruch 5, wobei der Krebs Leukämie, myelodysplastisches Syndrom, malingnes Lymphom, multiples Myelom, Magenkrebs, Kolonkrebs, Lungenkrebs, Brustkrebs, Keimzellenkrebs, Leberkrebs, Hautkrebs, Blasenkrebs, Prostatakrebs, Gebärmutterkrebs, Gehärmutterhalskrebs oder Eierstockkrebs ist.

7. Verwendung nach Anspruch 6, wobei das kationische Liposom ein Liposom ist, welches N-[1-(2,3-Dioleyloxy)propyl]-N,N,N-trimethylammoniumchlorid, N-[1-(2,3-Dioleyloxy)propylj-N,N,N-trimethylammoniummethylsulfat oder Dioctadecylamid-glycylspermin oder ein Gemisch davon mit einem neutralen Lipid umfasst.

8. Verwendung nach Anspruch 7, wobei das kationische Liposom Lipofektin ist.

9. Krebsantigenpeptid, umfassend die Aminosäuresequenz Arg Met Phe Pro Asn Ala Pro Tyr Leu (SEQ ID Nr. 5) und ein kationisches Liposom zur Behandlung von Patienten mit Krebs.

10. Krebsantigenpeptid und kationisches Liposom nach Anspruch 9, wobei der Krebs Leukämie, myelodysplastisches Syndrom, malingnes Lymphom, multiples Myelom, Magenkrebs, Kolonkrebs, Lungenkrebs, Brustkrebs, Keimzellenkrebs, Leberkrebs, Hautkrebs, Blasenkrebs, Prostatakrebs, Gebärmutterkrebs, Gebärmutterhalskrebs oder Eierstockkrebs ist.

11. Krebsantigenpeptid und kationisches Liposom nach einem der Ansprüche 9 oder 10, wobei das kationische Liposom ein Liposom ist, welches N-[1-(2,3-Dioleyloxy)propyl]-N,N,N-trimethylammoniumchlorid, N-[1-(2,3-Dioleyloxy)propyl]-N,N,N-trimethylammoniummethylsulfat oder Dioctadecylamid-glycylspermin oder ein Gemisch davon mit einem neutralen Lipid umfasst.

12. Krebsantigenpeptid und kationisches Liposom nach Anspruch 11, wobei das kationische Liposom Lipofektin ist.

**Revendications**

1. Vaccin anticancéreux comprenant le peptide antigène de cancer comprenant la séquence d'aminoacides Arg Met Phe Pro Asn Ala Pro Tyr Leu (SEQ ID NO : 5) et un liposome cationique.

2. Vaccin anticancéreux selon la revendication 1 où ledit cancer est la leucémie, le syndrome myélodysplasique, le lymphome malin, le myélome multiple, le cancer gastrique, le cancer du côlon, le cancer du poumon, le cancer du sein, le cancer des cellules germinales, le cancer du foie, le cancer de la peau, le cancer de la vessie, le cancer de la prostate, le cancer de l'utérus, le cancer du col ou le cancer ovarien.

3. Vaccin anticancéreux selon la revendication 1 ou 2 où ledit liposome cationique est un liposome comprenant du chlorure de N-[1-(2,3-dioléyloxy)propyl]-N,N,N-triméthylammonium, du méthylsulfate de N-[1-(2,3-dioléyloxy)propyl]-N,N,N-triméthylammonium ou de la dioctadecylamide-glycylspermine ; ou un mélange de ceux-ci avec un lipide neutre.

4. Vaccin anticancéreux selon la revendication 3 où ledit liposome cationique est la lipofectine.

5. Utilisation du peptide antigène de cancer comprenant la séquence d'aminoacides Arg Met Phe Pro Asn Ala Pro Tyr Leu (SEQ ID NO : 5) et d'un liposome cationique pour la production d'un vaccin anticancéreux.

6. Utilisation selon la revendication 5 où ledit cancer est la leucémie, le syndrome myélodysplasique, le lymphome malin, le myélome multiple, le cancer gastrique, le cancer du côlon, le cancer du poumon, le cancer du sein, le cancer des cellules germinales, le cancer du foie, le cancer de la peau, le cancer de la vessie, le cancer de la prostate, le cancer de l'utérus, le cancer du col ou le cancer ovarien.

7. Utilisation selon la revendication 6 où ledit liposome cationique est un liposome comprenant du chlorure de N-[1-(2,3-dioléyloxy)propyl]-N,N,N-trimethylammonium, du méthylsulfate de N-[1-(2,3-dioléyloxy)propyl]-N,N,N-triméthylammonium ou de la dioctadécylamide-glycylspermine ; ou un mélange de ceux-ci avec un lipide neutre.

8. Utilisation selon la revendication 7 où ledit liposome cationique est la lipofectine.

9. Peptide antigène de cancer comprenant la séquence d'aminoacides Arg Met Phe Pro Asn Ala Pro Tyr Leu (SEQ ID NO : 5) et liposome cationique pour traiter des patients ayant un cancer.

10. Peptide antigène de cancer et liposome cationique selon la revendication 9 où ledit cancer est la leucémie, le syndrome myélodysplasique, le lymphome malin, le myélome multiple, le cancer gastrique, le cancer du côlon, le cancer du poumon, le cancer du sein, le cancer des cellules germinales, le cancer du foie, le cancer de la peau, le cancer de la vessie, le cancer de la prostate, le cancer de l'utérus, le cancer du col ou le cancer ovarien.

11. Peptide antigène de cancer et liposome cationique selon l'une quelconque des revendications 9 ou 10 où ledit liposome cationique est un liposome comprenant du chlorure de N-[1-(2,3-dioléyloxy)propyl]-N,N,N-triméthylammonium, du méthylsulfate de N-[1-(2,3-dioléyloxy)propyl]-N,N,N-triméthylammonium ou de la dioctadéeylamide-glycylspermine; ou un mélange de ceux-ci avec un lipide neutre.

12. Peptide antigène de cancer et liposome cationique selon la revendication 11 où ledit liposome cationique est la lipofectine.

Fig.1

# Fig.2

EP 2 014 300 B1

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 9104627 A
- JP 9191635 A
- JP 2001199449 A

**Non-patent literature cited in the description**

- *Curr. Opin. Immuno.,* 1993, vol. 5, 709
- *Curr. Opin. Immunol.,* 1993, vol. 5, 719
- *Cell,* 1995, vol. 82, 13
- *Immunol. Rev.,* 1995, vol. 146, 167
- *Curr. Opin. Immunol.,* 1993, vol. 5, 709
- **Gessler, M. et al.** *Nature,* 1990, vol. 343, 774-778
- *Mol. Cell. Biol.,* 1991, vol. 11, 1707
- *Rinsho Menneki,* 2000, vol. 34 (6), 842-847
- *Immunogenetics,* 1995, vol. 41, 178-228
- **Mol. Cell. Biol.** *Mol. Cell. Biol.,* 1991, vol. 11, 1707-1712

SEQUENCE LISTING

<110> CHUGAI SEIYAKU KABUSHIKI KAISHA

<120> Cancer Vaccine Containing Cancer Antigen Based on Tumor Suppressor Gene WT1 Product and Cationic Liposomes

<130> P019217EPA

<150> JP 2001-199449
<151> 2001-06-29

<160> 9

<170> PatentIn version 3.5

<210> 1
<211> 449
<212> PRT
<213> Mus musculus

<400> 1

```
Met Gly Ser Asp Val Arg Asp Leu Asn Ala Leu Leu Pro Ala Val Ser
                  5                  10                  15
Ser Leu Gly Gly Gly Gly Gly Gly Cys Gly Leu Pro Val Ser Gly Ala
              20                  25                  30
Arg Gln Trp Ala Pro Val Leu Asp Phe Ala Pro Pro Gly Ala Ser Ala
          35                  40                  45
Tyr Gly Ser Leu Gly Gly Pro Ala Pro Pro Pro Ala Pro Pro Pro Pro
      50                  55                  60
Pro Pro Pro Pro His Ser Phe Ile Lys Gln Glu Pro Ser Trp Gly Gly
  65                  70                  75                  80
Ala Glu Pro His Glu Glu Gln Cys Leu Ser Ala Phe Thr Leu His Phe
                  85                  90                  95
Ser Gly Gln Phe Thr Gly Thr Ala Gly Ala Cys Arg Tyr Gly Pro Phe
              100                 105                 110
Gly Pro Pro Pro Pro Ser Gln Ala Ser Ser Gly Gln Ala Arg Met Phe
          115                 120                 125
Pro Asn Ala Pro Tyr Leu Pro Ser Cys Leu Glu Ser Gln Pro Thr Ile
      130                 135                 140
Arg Asn Gln Gly Tyr Ser Thr Val Thr Phe Asp Gly Ala Pro Ser Tyr
  145                 150                 155                 160
Gly His Thr Pro Ser His His Ala Ala Gln Phe Pro Asn His Ser Phe
              165                 170                 175
Lys His Glu Asp Pro Met Gly Gln Gln Gly Ser Leu Gly Glu Gln Gln
          180                 185                 190
Tyr Ser Val Pro Pro Pro Val Tyr Gly Cys His Thr Pro Thr Asp Ser
      195                 200                 205
Cys Thr Gly Ser Gln Ala Leu Leu Leu Arg Thr Pro Tyr Ser Ser Asp
  210                 215                 220
Asn Leu Tyr Gln Met Thr Ser Gln Leu Glu Cys Met Thr Trp Asn Gln
  225                 230                 235                 240
Met Asn Leu Gly Ala Thr Leu Lys Gly Met Ala Ala Gly Ser Ser Ser
              245                 250                 255
Ser Val Lys Trp Thr Glu Gly Gln Ser Asn His Gly Ile Gly Tyr Glu
          260                 265                 270
Ser Glu Asn His Thr Ala Pro Ile Leu Cys Gly Ala Gln Tyr Arg Ile
      275                 280                 285
His Thr His Gly Val Phe Arg Gly Ile Gln Asp Val Arg Arg Val Ser
  290                 295                 300
Gly Val Ala Pro Thr Leu Val Arg Ser Ala Ser Glu Thr Ser Glu Lys
  305                 310                 315                 320
Arg Pro Phe Met Cys Ala Tyr Pro Gly Cys Asn Lys Arg Tyr Phe Lys
```

```
                          325                      330                      335
      Leu Ser His Leu Gln Met His Ser Arg Lys His Thr Gly Glu Lys Pro
                  340
      Tyr Gln Cys Asp Phe Lys Asp Cys Glu Arg Arg Phe Ser Arg Ser Asp
                  355                      360                      365
      Gln Leu Lys Arg His Gln Arg Arg His Thr Gly Val Lys Pro Phe Gln
          370                      375                      380
      Cys Lys Thr Cys Gln Arg Lys Phe Ser Arg Ser Asp His Leu Lys Thr
      385                      390                      395                      400
      His Thr Arg Thr His Thr Gly Lys Thr Ser Glu Lys Pro Phe Ser Cys
                  405                      410                      415
      Arg Trp His Ser Cys Gln Lys Lys Phe Ala Arg Ser Asp Glu Leu Val
                  420                      425                      430
      Arg His His Asn Met His Gln Arg Asn Met Thr Lys Leu His Val Ala
                  435                      440                      445
      Leu
      449
```

<210> 2
<211> 449
<212> PRT
<213> Homo sapiens

<400> 2

```
Met Gly Ser Asp Val Arg Asp Leu Asn Ala Leu Leu Pro Ala Val Pro
                5                   10                  15
Ser Leu Gly Gly Gly Gly Gly Cys Ala Leu Pro Val Ser Gly Ala Ala
                20                  25                  30
Gln Trp Ala Pro Val Leu Asp Phe Ala Pro Pro Gly Ala Ser Ala Tyr
        35                  40                  45
Gly Ser Leu Gly Gly Pro Ala Pro Pro Pro Ala Pro Pro Pro Pro Pro
    50                  55                  60
Pro Pro Pro Pro His Ser Phe Ile Lys Gln Glu Pro Ser Trp Gly Gly
65                  70                  75                  80
Ala Glu Pro His Glu Glu Gln Cys Leu Ser Ala Phe Thr Val His Phe
                85                  90                  95
Ser Gly Gln Phe Thr Gly Thr Ala Gly Ala Cys Arg Tyr Gly Pro Phe
            100                 105                 110
Gly Pro Pro Pro Pro Ser Gln Ala Ser Ser Gly Gln Ala Arg Met Phe
        115                 120                 125
Pro Asn Ala Pro Tyr Leu Pro Ser Cys Leu Glu Ser Gln Pro Ala Ile
    130                 135                 140
Arg Asn Gln Gly Tyr Ser Thr Val Thr Phe Asp Gly Thr Pro Ser Tyr
145                 150                 155                 160
Gly His Thr Pro Ser His His Ala Ala Gln Phe Pro Asn His Ser Phe
            165                 170                 175
Lys His Glu Asp Pro Met Gly Gln Gln Gly Ser Leu Gly Glu Gln Gln
            180                 185                 190
Tyr Ser Val Pro Pro Pro Val Tyr Gly Cys His Thr Pro Thr Asp Ser
    195                 200                 205
Cys Thr Gly Ser Gln Ala Leu Leu Leu Arg Thr Pro Tyr Ser Ser Asp
    210                 215                 220
Asn Leu Tyr Gln Met Thr Ser Gln Leu Glu Cys Met Thr Trp Asn Gln
225                 230                 235                 240
Met Asn Leu Gly Ala Thr Leu Lys Gly Val Ala Ala Gly Ser Ser Ser
            245                 250                 255
Ser Val Lys Trp Thr Glu Gly Gln Ser Asn His Ser Thr Gly Tyr Glu
            260                 265                 270
Ser Asp Asn His Thr Thr Pro Ile Leu Cys Gly Ala Gln Tyr Arg Ile
    275                 280                 285
His Thr His Gly Val Phe Arg Gly Ile Gln Asp Val Arg Arg Val Pro
    290                 295                 300
```

```
Gly Val Ala Pro Thr Leu Val Arg Ser Ala Ser Glu Thr Ser Glu Lys
305                 310                 315                 320
Arg Pro Phe Met Cys Ala Tyr Pro Gly Cys Asn Lys Arg Tyr Phe Lys
            325                 330                 335
Leu Ser His Leu Gln Met His Ser Arg Lys His Thr Gly Glu Lys Pro
        340                 345                 350
Tyr Gln Cys Asp Phe Lys Asp Cys Glu Arg Arg Phe Ser Arg Ser Asp
        355                 360                 365
Gln Leu Lys Arg His Gln Arg Arg His Thr Gly Val Lys Pro Phe Gln
    370                 375                 380
Cys Lys Thr Cys Gln Arg Lys Phe Ser Arg Ser Asp His Leu Lys Thr
385                 390                 395                 400
His Thr Arg Thr His Thr Gly Lys Thr Ser Glu Lys Pro Phe Ser Cys
            405                 410                 415
Arg Trp Pro Ser Cys Gln Lys Lys Phe Ala Arg Ser Asp Glu Leu Val
            420                 425                 430
Arg His His Asn Met His Gln Arg Asn Met Thr Lys Leu Gln Leu Ala
    435                 440                 445
```

Leu 449

<210> 3
<211> 8

8

<212> PRT
<213> Artificial Sequence

<220>
<223> Synthetic Peptide

<400> 3

```
Gly Ala Ser Ala Tyr Gly Ser Leu
1               5
```

<210> 4
<211> 8
<212> PRT
<213> Artificial Sequence

<220>
<223> Synthetic Peptide

<400> 4

```
Cys Asn Lys Arg Tyr Phe Lys Leu
1               5
```

<210> 5
<211> 9
<212> PRT
<213> Artificial Sequence

<220>
<223> Synthetic Peptide

<400> 5

```
Arg Met Phe Pro Asn Ala Pro Tyr Leu
1               5
```

<210> 6
<211> 9
<212> PRT
<213> Artificial Sequence

<220>
<223> Synthetic Peptide

<400> 6

```
Tyr Ser Ser Asp Asn Leu Tyr Gln Met
1               5
```

<210> 7
<211> 9
<212> PRT
<213> Artificial Sequence

<220>
<223> Synthetic Peptide

<400> 7

```
Cys Met Thr Trp Asn Gln Met Asn Leu
1               5
```

<210> 8
<211> 9
<212> PRT
<213> Artificial Sequence

<220>
<223> Synthetic Peptide

<400> 8

```
Ser Leu Gly Glu Gln Gln Tyr Ser Val
1               5
```

<210> 9
<211> 9
<212> PRT
<213> Artificial Sequence

<220>
<223> Synthetic Peptide

<400> 9

```
Cys Tyr Thr Trp Asn Gln Met Asn Leu
1               5
```